(19) Europäisches Patentamt
European Patent Office
Office européen des brevets

(11) Veröffentlichungsnummer : **0 090 359**
**B1**

(12) # EUROPÄISCHE PATENTSCHRIFT

(45) Veröffentlichungstag der Patentschrift : **21.12.88**

(51) Int. Cl.⁴ : **C 12 Q 1/38, C 12 N 9/64**

(21) Anmeldenummer : **83102949.1**

(22) Anmeldetag : **24.03.83**

(54) **Verfahren zum Nachweis des Vorliegens einer Allergie und zum spezifischen Nachweis des für die Allergie verantwortlichen Allergens.**

(30) Priorität : **26.03.82 DE 3211254**

(43) Veröffentlichungstag der Anmeldung : **05.10.83 Patentblatt 83/40**

(45) Bekanntmachung des Hinweises auf die Patenterteilung : **21.12.88 Patentblatt 88/51**

(84) Benannte Vertragsstaaten : **AT BE CH DE FR GB IT LI LU NL SE**

(56) Entgegenhaltungen :
**EP–A– 0 014 929**
**DD–A– 128 691**
**DE–A– 3 147 763**
**FR–A– 2 315 695**
**CHEMICAL ABSTRACTS Band 60, Nr. 2, 20 Januar 1964,Columbus, Ohio, USA; C.A. MOUNTER et al. "Proteases of human leukocytes", Spalte 1987 & Blood Band 15, 1960, Seiten 52-59**
**J.Clin.Invest., vol. 64, August 1979, pp. 457-465, Römpp 3651**

(73) Patentinhaber : **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

(72) Erfinder : **Wilhelms, Otto-Henning, Dr.**
**Odenwaldstrasse 25/2**
**D-6941 Weinheim-Rittenweier (DE)**
Erfinder : **Stahl, Peter, Dr.**
**Hirtenstrasse 12**
**D-8131 Bernried (DE)**
Erfinder : **Wunderwald, Peter, Dr.**
**Schulstrasse 6**
**D-8121 Haunshofen (DE)**

(74) Vertreter : **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel**
**Postfach 860820**
**D-8000 München 86 (DE)**

**EP 0 090 359 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zum Nachweis des Vorliegens einer Allergie und zum spezifischen Nachweis des für die Allergie verantwortlichen Allergens sowie eine neue Protease, deren Aktivität im Rahmen des Verfahrens der Erfindung bestimmt wird.

Bei Allergikern finden sich im Blut erhöhte IgE-Spiegel. IgE findet sich außerdem gebunden an Mastzellen und Basophilen. Die allergische Reaktion wird eingeleitet durch Bindung eines Allergens an das dafür spezifische an Mastzellen bzw. Basophile gebundene IgE.

Vielfach wird angenommen, daß diese Bindung des stimulierenden Allergens an IgE über die sog. « Verbrückung » benachbarter IgE in komplexer Reaktion die Degranulation von basophilen Leukozyten bewirkt. Die Degranulation setzt bekanntermaßen Histamin, eine Kallikrein-ähnliche Protease sowie andere Substanzen frei.

Für die Diagnostik allergischer Erkrankungen gewinnen neben den Patienten belastenden und mit einem gewissen Risiko behafteten in vivo-Tests zunehmend in vitro-Tests Bedeutung. Kommerziell erhältliche in vitro-Tests erfassen entweder Gesamt-IgE (allgemeiner unspezifischer Nachweis des Vorliegens einer Allergie) oder spezifisches IgE (Nachweis der Spezifität des vorhandenen IgE, das für die Allergie verantwortlich ist). Vorteile der genannten in vitro-Tests sind relativ rasche Durchführung und geringe Belastung des Patienten ; Nachteile sind, daß die IgE-Konzentration im Serum nicht notwendigerweise ein Parameter für die Reaktionsfähigkeit von Mastzellen und Basophilen (Freisetzbarkeit verschiedener Mediatoren) ist, und daß die Erfassung des spezifischen IgE trägergebundene Allergene erfordert, die aufgrund ihrer Bindung an den Träger verändert sind. Dementsprechend berichtet die Literatur über falschnegative Resultate.

Man hat schon versucht, diese Nachteile durch celluläre Tests zu beseitigen, bei denen die Stimulation von sensitiven Zellen mit Allergen in vitro erfolgt und die allergenstimulierte Histamin-Freisetzung gemessen wird. Diese Methode erfaßt den eigentlichen Vorgang, der die allergische Reaktion bedingt. Ein Nachteil dieser Verfahren ist jedoch darin zu sehen, daß die Histamin-Analytik nur mit größerem Aufwand durchzuführen und störanfällig ist.

Ferner wurde bereits die Allergen-spezifische Freisetzung einer Kallikrein-ähnlichen Protease aus Leukozyten beschrieben (J. Clin. Invert., Vol. 64, August 1979, pp. 457 to 465). Diese Protease besitzt ein pH-Optimum bei 8,5, eine spezifische Hemmbarkeit durch Aprotinin $I_{50}$ von ca. 50 µg/ml und 11 % Hemmung durch 0,1 mol/l STI (Sojabohnen-Trypsin-Inhibitor). Die Bestimmung dieses Enzyms erfolgt radiochemisch mit $^3$H-TAME (Tosyl-Arginyl-Methylester), da nur sehr wenig Aktivität freigesetzt wird. Die Freisetzung dieser Protease soll auch unspezifisch mit Anti-IgE oder spezifisch mit Allergen erfolgen können.

Der Erfindung liegt die Aufgabe zugrunde, ein Verfahren zu schaffen, das die Spezifität der cellulären Reaktion mit einer relativ einfachen Durchführung und hoher Empfindlichkeit verbindet.

Diese Aufgabe wird erfindungsgemäß gelöst durch ein Verfahren zum Nachweis des Vorliegens einer Allergie und zum spezifischen Nachweis des für die Allergie verantwortlichen Allergens auf Basis des cellulären Prinzips, dadurch gekennzeichnet, daß man Leukozyten der zu untersuchenden Probe mit einem Allergen oder einem anderen Stimulationsfaktor der Degranulation, wie Anti-IgE, in wässrigem Medium inkubiert, dann die Leukozyten abtrennt und die verbleibende Lösung mit einer Verbindung der allgemeinen Formel

$$Y—X—C$$

in der Y eine Aminosäure oder ein Peptid mit 2 oder 3 Aminosäuren, wobei die Aminosäuren ggf. am Aminoende eine übliche Schutzgruppe tragen können, X Arginin oder Lysin und C einen chromogenen Rest bedeuten, in Gegenwart eines bei pH 6,3 bis 8,0 wirksamen Puffers inkubiert und die Freisetzung des chromogenen Restes mißt.

Die Erfindung beruht auf der überraschenden Auffindung einer neuen Protease, die aus Leukozyten unspezifisch mit Anti-IgE oder spezifisch mit Allergen freigesetzt wird, wenn es sich um Leukozyten von Atopikern, d. h. allergisch sensibilisierten Personen, handelt. Die neue Protease, die in ihren Eigenschaften deutlich von der bekannten Kallikrein-ähnlichen Protease aus Leukozyten verschieden ist, spaltet chromogene Reste tragende Peptide mit guter Aktivität. Da Leukozyten von Atopikern eine 5 bis 15fach höhere Ausschüttung an dieser Protease ergeben im Vergleich zu Leukozyten von gesunden Personen, läßt sich hier durch Bestimmung der Aktivität dieser Protease ein empfindlicher und schneller Allergietest hoher Spezifität aufbauen.

Die neue Protease aus Leukozyten zeichnet sich durch eine spezifische Hemmbarkeit durch Aprotinin $I_{50}$ von 1 mg/ml, eine spezifische Hemmbarkeit durch STI von 50 bis 100 % bei 0,05 mMol/l aus. Die Protease ist bis zu etwa 100 Stunden nach der Freisetzung stabil, so daß auch die Messung erhebliche Zeit nach der Blutentnahme durchgeführt werden kann, nachdem auch die Zellen selbst relativ stabil sind. Sie weist ein breites pH-Optimum zwischen etwa 6,8 und 8,2 auf, bestimmt unter den im Beispiel 1 angegebenen Bedingungen.

Das Substrat der Leukozytenprotease mit der allgemeinen Formel

Y—X—C

enthält als C einen der bekannten chromogenen Reste, welcher durch die neue Leukozytenprotease abgespalten und dann beim erfindungsgemäßen Verfahren quantitativ bestimmt wird. Bevorzugt ist C ein p-Nitranilinrest. Als chromogener Rest wird im Rahmen der Erfindung ein Rest verstanden, der nach Abspaltung, gegebenenfalls durch Umsetzung mit einer weiteren Verbindung, in eine gefärbte Verbindung überführt werden kann oder selbst gefärbt ist. Der Rest Y enthält vorzugsweise die Sequenz Gly-Pro.

Besonders bevorzugte Verbindungen der allgemeinen Formel Y—X—C sind Tosyl-Glycyl-Prolyl-Arginin-p-Nitranilid-Acetat (im Handel erhältlich unter der Bezeichnung Chromozym TH) sowie die entsprechende Verbindung, in der Arginin durch Lysin ersetzt ist. Die Verbindungen Y—X—C können auch in trägergebundener Form verwendet werden.

Das erfindungsgemäße Verfahren weist eine ungewöhnlich breite Anwendbarkeit auf. So wurde beispielsweise mit seiner Anwendung eine Ampizillin-Allergie nachgewiesen, was besonders überraschend ist, da dieser Befund nicht mit den bisher bekannten Auslösemechanismen der allergischen Reaktion zu erklären ist. Ebenso konnte eine Metallallergie gegenüber Ni- bzw. Cr-Ionen nachgewiesen werden.

Um erfindungsgemäß eine Allergie nachweisen zu können, ist es lediglich notwendig, gewaschene Leukozyten der Versuchsperson zu beschaffen, diese mit einem spezifischen Allergen oder einem unspezifischen anderen Stimulationsfaktor der Degranulation, wie z. B. Anti-IgE zu inkubieren, nach der Inkubation die Leukozyten aus dem Überstand abzutrennen, das Substrat und den Puffer zuzusetzen und nach einiger Zeit das freigesetzte Chromogen zu bestimmen. Verwendet man hierbei für die Inkubation ein spezifisches Allergen, so erhält man eine erhöhte Ausschüttung der erfindungsgemäßen Leukozyten-Protease nur dann, wenn die Leukozyten gegen dieses Allergen sensibilisiert sind.

Unspezifische Stimulationsfaktoren der Degranulation außer Anti-IgE wurden bereits in größerer Zahl beschrieben, z. B. in A. L. DeWeck : Perspective in Allergy Intern. Congress of Chemotherapy, Florence, 1981.

Die Herstellung der für das erfindungsgemäße Verfahren verwendeten gereinigten Leukozyten erfolgt nach dem Fachmann bekannten Methoden, die hier nicht näher beschrieben werden müssen. Sowohl die Dextranmethode Prof. Dr. H. Friemel : Immunologische Arbeitsmethoden Gustav Fischer Verlag, Stuttgart, 1980 als auch die Ficollmethode N. R. Rose, H. Friedmann : Manual of Clinical Immunology ASM, Washington, D. C. 1980 können für die Granulozyten-Anreicherung verwendet werden. Die erhaltene Zellsuspension wird zweckmäßig mit geeignetem Puffer versetzt, bei etwa 300 bis 800 g zentrifugiert und das Sediment in frischem Puffer suspendiert. Die Waschung kann ggf. wiederholt durchgeführt werden. Für den Bestimmungsansatz sollte die Zellsuspension zweckmaßig im Bereich von $10^6$ bis $10^8$ Zellen pro ml liegen. Anstelle von Leukozyten können bei der Erfindung auch Mastzellen verwendet werden.

Als Puffer kann im Rahmen der Erfindung jeder im angegebenen pH-Bereich wirksame Puffer verwendet werden. Gut hat sich Trispuffer bewährt.

Der erste Inkubationsschritt, bei dem die Leukozyten mit z. B. Anti-IgE bzw. einem Allergen umgesetzt werden, hängt in seiner Dauer von der angewendeten Temperatur ab. Bei 37 °C inkubiert man zweckmäßig etwa 60 Minuten. Die Abtrennung der Leukozyten nach der Inkubation erfolgt nach bekannten Methoden, beispielsweise durch 10 Minuten zentrifugieren bei 30 000 g. Der dabei erhaltene Zentrifugationsüberstand wird direkt mit dem Substrat der allgemeinen Formel Y—X—C versetzt und erneut inkubiert. Zur Bestimmung des Meßwerts wird vorzugsweise eine Vergleichsprobe mitgeführt, welche anstelle der Anti-IgE bzw. Allergenlösung eine entsprechende Menge Pufferlösung zugesetzt erhält.

Die Messung des Chromogens erfolgt mit den dem Fachmann bekannten optischen Methoden, entweder im Sichtbaren oder im U. V.-Bereich, in Abhängigkeit von der Farbe des abgespaltenen Restes.

Gemäß einer besonderen Ausführungsform des erfindungsgemäßen Verfahrens wird es möglich, erstmals auch die Bestimmung eines latenten Allergierisikos durchzuführen. Bei dieser weiterentwickelten Modifikation des Verfahrens wird das zu testende Zellmaterial erst mit Immunoglobulin E (IgE) inkubiert. Hierdurch wird eine Sensibilisierung des Zellmaterials bewirkt, die zur Folge hat, daß unter den Bedingungen des erfindungsgemäßen Verfahrens ein Ergebnis wie beim Vorliegen einer Allergie erhalten wird. Mit dem selben Zellmaterial erhält man bei Weglassen der Vorinkubation mit IgE Ergebnisse wie bei einem Nichtallergiker. Es ist daher möglich, auf diese Weise zwischen Fehlen einer Allergie, latenter Allergie und akuter Allergie zu unterscheiden.

Durch das erfindungsgemäße Verfahren wird eine einfache, rasche und spezifische Bestimmungsmethode geschaffen, die sowohl das Vorliegen einer Allergie generell als auch den Nachweis des spezifischen Allergens gestattet, ohne den Patienten hierüber zu belasten, wie dies bei den in vivo-Tests in Kauf genommen werden muß. Das Verfahren der Erfindung verbindet daher die Vorteile der bekannten in vivo-Tests mit den Vorteilen der in vitro-Tests. Die folgenden Beispiele erläutern die Erfindung weiter.

## Beispiel 1

Probe von gesundem Spender

### 1.1 Leukozyten-Gewinnung

60 ml venöses Blut eines gesunden Spenders wurde mit 30 ml Dextran-Gemisch (Dextran mit mittlerem MG 75000 2 %; d-Glucose 2 %; EDTA 20 mmol/l ; Gelatine 0,07 %) vorsichtig in einem Polyäthylen-Becherglas gemischt und in einen silikonisierten Scheidetrichter überführt. Nach 60 bis 90 Minuten Stehenlassen bei Raumtemperatur wurde der die Leukozyten enthaltende Überstand (ca. 50 ml) abgetrennt und mit 50 ml Tris-Puffer-Gemisch, pH 7,6 (Tris 22 mmol/l ; NaCl 0,12 mol/l ; KCl 5 mmol/l ; Gelatine 0,05 %; EDTA 1 mmol/l ; D-Glucose 0,1 %) versetzt und 15 Minuten bei 800 g zentrifugiert. Nach Dekantieren wurden die Zellen 2mal gewaschen, indem sie jeweils mit nochmals 50 ml des gleichen Puffergemisches, pH 7,6, suspendiert und jeweils 15 Minuten bei 800 g zentrifugiert wurden. Die Zellen wurden in 10 ml des gleichen Puffergemisches suspendiert.

### 1.2 Protease-Freisetzung

2mal 0,9 ml Zellsuspension wurde in je ein Polyäthylen-Reagenzglas überführt und mit 0,4 ml Tris-Puffer-Gemisch, pH 7,6, im Wasserbad auf 37 °C gebracht. In eine Probe wurden 0,1 ml Anti-IgE-Lösung (Behring-Werke, Nr. OTNP 04/05, mit Tris-Puffer-Gemisch, pH 7,6, 1 : 500 verdünnt) gegeben, in die andere Probe — die Kontrolle — wurde 0,1 ml Tris-Puffer-Gemisch, pH 7,6, pipettiert und zu beiden Proben 0,1 ml $CaCl_2$-Lösung (4 mmol/l in isotonischer NaCl-Lösung, pH 7,6) zugefügt. Die beiden Proben wurden 60 Minuten bei 37° gehalten, dann mit je 0,5 ml eiskalter EDTA-Lösung (0,1 mol/l in isotonischer NaCl, pH 7,6) versetzt und 10 Minuten bei 4 °C bei 30 000 g zentrifugiert und der Überstand gleich nach Auslaufen der Zentrifuge abpipettiert.

### 1.3 Protease-Testung

Zu 0,5 ml des Überstandes wurden 0,5 ml 1,5 mmol/l Chromozym TH-Lösung (1 Flasche BM 199664 in 5 ml $H_2O$ lösen) gegeben. Die Extinktionsdifferenz zwischen den beiden Proben wurde nach 1, 2 und 3 Stunden bei 405 nm bestimmt.

### 1.4 Ergebnis

In den 3 Stunden wurde in der unbehandelten Probe eine konstante Extinktionszunahme von $\Delta E/h = 0{,}020$ gefunden, in der mit Anti-IgE behandelten Probe ein $\Delta E/h$ von 0,065. Anti-IgE-Behandlung der Zellen führte also zu einer Extinktionszunahme von $\Delta E_{405nm/h} = 0{,}045$.

### Anmerkung

In Blut von nicht allergischen Spendern wurde mit Anti-IgE mit der Methode dieses Beispiels kein Wert über $\Delta E/h = 0{,}1$ beobachtet. In einzelnen Fällen wurde über 20 Stunden konstante Extinktionszunahme beobachtet.

## Beispiel 2

Probe eines Spenders mit nachgewiesener Allergie auf Ampicillin

### 2.1 Nachweis des Vorhandenseins einer Allergie durch Anti-IgE-Stimulierung

Aus 0,9 ml der entsprechend Punkt 1.1 gewonnenen Leukozyten-Suspension eines auf Ampicillin allergischen Spenders entsprechend 1.2 durch Anti-IgE freigesetzte Protease (Meßwert : $\Delta E/h = 0{,}805$) ergab gegenüber der nicht mit Anti-IgE stimulierten Vergleichsprobe (Meßwert : $\Delta E/h = 0{,}085$) eine zusätzliche Extinktionsveränderung nach $\Delta E/h = 0{,}720$ während der ersten drei Stunden nach Start der Reaktion mit Chromozym TH.

Aus 1 ml Blut wurden somit ca. 1,6 mU Chromozym TH hydrolysierende Aktivität freigesetzt.

### 2.2 Nachweis des Vorhandenseins einer Ampicillin-Allergie durch Ampicillin-Stimulierung

0,9 ml der entsprechend Beispiel 1.1 gewonnenen Leukozyten-Suspension wurden entsprechend 1.2 stimuliert, jedoch wurden statt 0,1 ml Anti-IgE-Lösung 0,1 ml Ampicillin/Serumalbumin-Lösung (Ampicillin 5 mg/ml, Humanserumalbumin 5 mg/ml) zur Freisetzung der Protease eingesetzt. Die durch Ampicillin freigesetzte Protease (Meßwert : $\Delta E/h = 0{,}893$) ergab gegenüber der nicht mit Ampicillin behandelten Kontrolle (Meßwert : $\Delta E/h = 0{,}335$) eine zusätzliche Extinktionsveränderung von $\Delta E/h = 0{,}558$.

Anmerkung

Serumalbumin ist zur Freisetzung der Protease mit Ampicillin nicht notwendig.

Beispiel 3

CBO-Gly-Pro-Arg-pNA als Substrat

In 2mal 0,9 ml entsprechend 1.1 gewonnener Leukozyten-Suspension aus Blut eines gesunden Spenders wurde entsprechend 1.2 Protease nach Anti-IgE-Stimulation als Zentrifugationsüberstand gewonnen. Die Testung der Protease erfolgt wie in 1.3, jedoch wurde in einer Probe statt Chromozym TH eine äquimolare Menge CBO-Gly-Pro-Arg-pNA eingesetzt. Innerhalb der ersten 3 Stunden wurden gleiche $\Delta E/h$ ($\Delta E/h = 0{,}067$) gemessen.

Beispiel 4

Tos-Gly-Pro-Lys-pNA (Chromozym PL) als Substrat

Die Aktivität von zwei entsprechend 1 parallel behandelten Proben wurde mit Chromozym TH bzw. einer äquimolaren Menge Chromozym PL bestimmt. Mit Chromozym PL wurde $\Delta E/h = 0{,}052$, mit Chromozym TH$\Delta E/h = 0{,}048$ gefunden.

Beispiel 5

Phe-Pip-Arg-pNA als Substrat

Die Protease-Aktivität von zwei entsprechend 1 parallel behandelten Proben wurde mit Chromozym TH bzw. einer äquimolaren Menge S 2238 bestimmt. Mit S 2238 wurden $\Delta E/h = 0{,}037$, mit Chromozym TH$\Delta E/h = 0{,}048$ bestimmt.

Beispiel 6

Die Durchführung erfolgt wie in Beispiel 1 beschrieben.

Testpersonen

I. Allergiker : Allergische Symptome (tränende Augen, Niesen usw.) bei Betreten eines Pferdestalles oder in der Nähe eines erhitzten Pferdes. Vermutetes Allergen : Pferdestaub.

II. Nichtallergiker : Keine allergischen Symptome bekannt.

Test

Zellkonzentration

Allergiker : $5 \times 10^7$ Zellen/ml
Nichtallergiker : $4 \times 10^7$ Zellen/ml
Auslösung der Degranulationsreaktion mit
a) anti IgE 1 : 500 verdünnt → 0,1 ml/1,5 ml Test
b) Pferdeepithelien der Fa. Allergopharm 1 : 100 verdünnt.
Verdünnungen : 1 : 100
1 : 1 000
1 : 10 000 → 0,1 ml/1,5 ml Test

Ergebnisse

1. Unspezifische Auslösung der allergischen Reaktion durch Anti IgE, d. h. Vorliegen einer Allergie :

Allergiker : $\Delta E_{405}/h = 1{,}344$
Nichtallergiker : $\Delta E_{405}/h = 0{,}038$

2. Spezifische Auslösung der allergischen Reaktion durch Pferdeepithelien-Allergen bei Allergiker I :

Pferdeepithelien-Allergen
1 : 100 verdünnt : $\Delta E_{405}/h = 1{,}122$

1 : 1 000 verdünnt : $\Delta E_{405}/h = 1{,}611$
1 : 10 000 verdünnt : $\Delta E_{405}/h = 1{,}598$

Beispiel 7

7.1 Leukozytengewinnung

60 ml frisches venöses Vollblut von gesunden Spendern oder Atopikern werden in einen 100 ml Erlenmeyer-Kolben (Polyäthylen) gegeben, in dem ein Gemisch aus 12,5 ml Dextranlösung (mittleres Mol-Gewicht 75 000), 12,5 ml D-Glucoselösung (6 %ig) und 6,0 ml EDTA-Lösung (100 mM) sowie 2,5 ml Gelatine-Lösung (2 %ig) vorgelegt wurde.

Nach Durchmischen werden Portionen von ca. jeweils 20 ml in Polypropylen-Serum — Plasmafilter (Monovetten, Fa. Sarstedt, Best.-Nr. 53.422 ; 25 ml) überführt und anschließend für ca. 60 bis 90 Minuten bei Raumtemperatur stehen gelassen.

Danach wird der Plasmaüberstand (insgesamt ca. 45 bis 50 ml) vom Erythrozyten-Sediment getrennt. 100 µl des Überstands werden für die Zellzählung (Leukozyten und Erythrozyten ; « coulter counter ») entnommen. Anschließend wird der Überstand mit eiskaltem Tris-Puffer (6 mM), pH 7,6 + EDTA (1 mM) + Gelatine (0,05 %) ca. 1 : 2 verdünnt, 15 Minuten bei 800 xg zentrifugiert, erneut mit demselben Puffer gewaschen und dann mit Tris-Puffer (6 mM) + Glucosezusatz (0,1 %ig) auf ca. 40 ml resuspendiert. Anschließend werden erneut 100 µl der Zellsuspension für eine erneute Zählung entnommen.

7.2 Reaktionsdurchführung

Je 0,9 ml der Leukozytenzellsuspension werden in ein Polyäthylen-Reagenzglas überführt, mit 0,4 ml Tris-Puffer-Gemisch mit Glucosezusatz, pH 7,6, im Wasserbad auf 37 °C gebracht und mit je 0,1 ml einer anti-IgE-Lösung (Behringwerke, Nr. OTNP 04/05, mit Tris-Puffergemisch, pH 7,6, 1 : 500 verdünnt), oder entsprechend mit je 0,1 ml Allergenlösung (Endkonzentration im Gesamtansatz berechnet auf 100, 10 und 1 µg/ml) versetzt und anschließend mit je 0,1 ml Kalziumchloridlösung (4 mM in isotonischer NaCl-Lösung, pH 7,6) versetzt.

Kontrollproben ohne anti-IgE bzw. Allergen-Stimulus erhalten entsprechend je 0,1 ml Tris-Puffergemisch, pH 7,6, zugesetzt.

Die Proben werden 60 Minuten bei 37 °C inkubiert, dann mit je 0,5 ml eiskalter EDTA-Lösung (10 mM/l in isotonischer NaCl-Lösung, pH 7,6) versetzt und 10 Minuten bei 4 °C bei 1 500 xg zentrifugiert. Der Überstand wird nach dem Auslaufen der Zentrifuge abpipettiert.

7.3 Analytik

Proteinase

Zur Messung der proteolytischen Aktivität werden je 0,5 ml des Zellüberstandes, der maximal 15 min/4 °C nach Abtrennung der Zellen aufbewahrt worden war, mit 0,7 ml Tris-Puffer, 0,075 M, pH 7,6 und 0,3 ml Chromozym TH-Lösung ($1{,}5 \times 10^{-3}$ M ; Boehringer Mannheim, Best.-Nr. 199664, jeweils 1 Flasche in 5 ml Wasser gelöst) gemischt. Zur Bestimmung der unspezifischen Substratspaltung durch

a) Proteinasen in den Allergenextrakten bzw.
b) im anti-IgE-Antiserum bzw.
c) durch den Arbeitspuffer

werden entsprechend Aliquote parallel zu den verschiedenen Zellproben mit Allergen bzw. mit anti-IgE-Zusatz angesetzt.

Unmittelbar danach werden die Ausgangsextinktionen der Proben bei 405 nm und Raumtemperatur gemessen. Anschließend werden die Proben über Nacht bei Raumtemperatur (20 bis 24 °C) stehen gelassen und nach ca. 20 Stunden die Extinktion erneut bei 405 nm abgelesen.

Als Maß für die spezifisch durch Allergen bzw. anti-IgE freisetzbare Proteinase dient die korrigierte Extinktion. Dieses ist der Probenmeßwert abzüglich der Extinktionen für

a) eine unspezifische Substratspaltung durch Allergen bzw. anti-IgE-Lösung sowie
b) der Extinktion in den « Kontrollproben ohne Allergen- bzw. anti-IgE-Stimulus » minus der Extinktion für die Probe von Chromozym TH + Puffer.

7.4 Atopiker

Leukozyten von einem Spender mit offensichtlichen Symptomen von Heuschnupfen wurden wie in 7.1 bis 7.3 beschrieben gewonnen, umgesetzt und analysiert. Nach einer Inkubation von 20 Stunden bei Raumtemperatur war die Extinktion deutlich angehoben von 0,030 (spontane, pufferinduzierte Freisetzung) auf 0,185 (Allergen I-induzierte Freisetzung). Das Ergebnis zeigt, daß zwar Allergen I (Raygras), nicht aber Allergen II (Alternaria) eine eindeutige Freisetzung von Proteinase aus den Leukozyten verursacht.

Ebenso war nach Stimulation mit antihuman-IgE eine eindeutige Freisetzung ($\Delta E_{405}$ : 0,197) feststellbar.

Beispiel 8

Nachweis latenter Allergie

Es wird das Testsystem gemäß Beispiel 1 angewendet. Die untersuchte Testzellsuspension (3 x $10^7$ Zellen/ml) wurde von einem latenten Allergiker als Spender erhalten. Mit dieser Zellsuspension wurde das Verfahren von Beispiel 1 wiederholt. In einem zweiten Ansatz wurde eine weitere Menge der Testzellsuspension 30 Minuten bei 37 °C mit einem mit IgE angereicherten Humanserum vorinkubiert. Danach wurden die Zellen zweimal mit Trispuffer gewaschen und danach wie im Beispiel 1 beschrieben, weiterbehandelt. Die Ergebnisse zeigt die nachstehende Tabelle :

| anti-IgE | | 1 Std. | 2 Std. | 3 Std. | 20 Std. |
|---|---|---|---|---|---|
| - | | 0,270 | 0,320 | 0,375 | 1,900 |
| + | | 0,390 | 0,500 | 0,640 | 3,500 |
| ΔE | | 0,120 | 0,180 | 0,265 | 1,600 |
| +IgE | - | 0,250 | 0,260 | 0,275 | 1,100 |
| +IgE | + | 0,580 | 0,930 | 1,300 | 7,100 |
| | ΔE | 0,330 | 0,670 | 1,025 | 6,000 |

Die Ergebnisse zeigen, daß bei Inkubation mit IgE eine Extinktionsdifferenz erhalten wird, die dem Vorliegen einer Allergie entspricht, während Abwesenheit von IgE die Extinktionsdifferenz keine Allergie anzeigt.

**Patentansprüche**

1. Verfahren zum Nachweis des Vorliegens einer Allergie und zum spezifischen Nachweis des für die Allergie verantwortlichen Allergens auf Basis des cellulären Prinzips, dadurch gekennzeichnet, daß man Leukozyten der zu untersuchenden Probe mit einem Allergen oder einem anderen Stimulationsfaktor der Degranulation, wie Anti-IgE, in wässrigem Medium inkubiert, dann die Leukozyten abtrennt und die verbleibende Lösung mit einer Verbindung der allgemeinen Formel

Y—X—C

in der Y eine Aminosäure oder ein Peptid mit 2 oder 3 Aminosäuren, wobei die Aminosäuren ggf. am Aminoende eine übliche Schutzgruppe tragen können, X Arginin oder Lysin und C einen chromogenen Rest bedeuten, in Gegenwart eines bei pH 6,3 bis 8,0 wirksamen Puffers inkubiert und die Freisetzung des chromogenen Restes mißt.

2. Verfahren nach Anspruch 1, dadurch gekennzeichnet, daß eine Verbindung der allgemeinen Formel verwendet wird, in welcher C eine p-Nitroanilingruppe darstellt.

3. Verfahren nach Anspruch 1 oder 2, dadurch gekennzeichnet, daß eine Verbindung der allgemeinen Formel verwendet wird, in der Y die Gruppe Gly-Pro enthält.

4. Verfahren nach einem der vorhergehenden Ansprüche, dadurch gekennzeichnet, daß man als Verbindung der allgemeinen Formel Tosyl-Glycyl-Prolyl-Arginin-p-Nitranilid-Acetat oder die entsprechende, anstelle von Arginin Lysin enthaltende Verbindung verwendet.

5. Verfahren nach einem der Ansprüche 1 bis 4, dadurch gekennzeichnet, daß die Verbindung der allgemeinen Formel

Y—X—C

in trägergebundener Form verwendet wird.

6. Verfahren nach einem der vorhergehenden Ansprüche, dadurch gekennzeichnet, daß in Gegenwart von Aprotinin inkubiert wird.

7. Abänderung des Verfahrens nach Anspruch 1 bis 6, dadurch gekennzeichnet, daß zum Nachweis des Vorliegens einer latenten Allergie und zum spezifischen Nachweis des für die latente Allergie verantwortlichen Allergens die Leukozyten der zu untersuchenden Probe mit IgE vorinkubiert und danach dem Verfahren eines der Ansprüche 1 bis 6 unterworfen werden.

**Claims**

1. Process for the detection of the presence of an allergy and for the specific detection of the allergen responsible for the allergy based on the cellular principle, characterised in that one incubates leukocytes of the sample to be investigated with an allergen or another stimulation factor of degranulation, such as anti-IgE, in aqueous medium, then separates off the leukocytes and incubates the remaining solution with a compound of the general formula

Y—X—C

in which Y signifies an amino acid or a peptide with 2 or 3 amino acids, whereby the amino acids can possibly carry a conventional protective group on the amino end, X arginine or lysine and C a chromogenic residue, in the presence of a buffer effective at pH 6.3 to 8.0 and measures the liberation of the chromogenic residue.

2. Process according to claim 1, characterised in that a compound of the general formula is used in which C represents a p-nitroaniline group.

3. Process according to claim 1 or 2, characterised in that a compound of the general formula is used in which Y contains the group Gly-Pro.

4. Process according to one of the preceding claims, characterised in that as compound of the general formula one uses tosyl-glycyl-prolyl-arginine-p-nitroanilide acetate or the corresponding compound containing lysine instead of arginine.

5. Process according to one of claims 1 to 4, characterised in that the compound of the general formula

Y—X—C

is used in carrier-bound form.

6. Process according to one of the preceding claims, characterised in that one incubates in the presence of aprotinin.

7. Modification of the process according to claim 1 to 6, characterised in that, for the detection of the presence of a latent allergy and for the specific detection of the allergen responsible for the latent allergy, the leukocytes of the sample to be investigated are preincubated with IgE and thereafter subjected to the process of one of claims 1 to 6.

**Revendications**

1. Procédé pour la détection d'une allergie et la caractérisation spécifique de l'allergène responsable de l'allergie sur la base du principe cellulaire, caractérisé en ce qu'on incube en milieu aqueux des leucocytes de l'échantillon à examiner avec un allergène ou un autre facteur de stimulation de la dégranulation, tel que l'anti-IgE, qu'on sépare ensuite les leucocytes, incube la solution résiduelle, en présence d'un tampon efficace à un pH de 6,3 à 8,0 avec un composé de formule générale

Y—X—C

dans laquelle Y représente un amino-acide ou un peptide avec 2 ou 3 amino-acides, les amino-acides pouvant éventuellement porter à l'extrémité amino un groupe protecteur classique, X représente l'arginine ou la lysine et C un reste chromogène et on mesure la libération du reste chromogène.

2. Procédé selon la revendication 1, caractérisé en ce qu'on utilise un composé selon la formule générale, dans laquelle C représente un groupe p-nitroaniline.

3. Procédé selon la revendication 1 ou 2, caractérisé en ce qu'on utilise un composé selon la formule générale, dans laquelle Y renferme le groupe Gly-Pro.

4. Procédé selon l'une des revendications précédentes, caractérisé en ce qu'on utilise comme composé selon la formule générale l'acétate de tosyl-glycyl-prolyl-arginine-p-nitranilide ou le composé correspondant contenant de la lysine à la place de l'arginine.

5. Procédé selon l'une des revendications 1 à 4, caractérisé en ce que le composé de formule générale

$$Y—X—C$$

est employé sous la forme fixée sur un support.

6. Procédé selon l'une des revendications précédentes, caractérisé en ce qu'on incube en présence d'aprotinine.

7. Modification du procédé selon les revendications 1 à 6, caractérisée en ce que, pour la mise en évidence d'une allergie latente et pour la détermination spécifique de l'allergène responsable de l'allergie latente, les leucocytes de l'échantillon à examiner sont pré-incubés avec de l'IgE et soumis ensuite au procédé d'une des revendications 1 à 6.